Europäisches Patentamt

European Patent Office

Office européen des brevets

(11) Publication number: **0 177 329 B1**

# EUROPEAN PATENT SPECIFICATION

(45) Date of publication of patent specification: **30.09.92**

(51) Int. Cl.⁵: **A61L 15/24**, A61L 15/64, A61K 9/70, A61M 37/00

(21) Application number: **85307000.1**

(22) Date of filing: **01.10.85**

(54) **Device for transdermal drug administration.**

(30) Priority: **05.10.84 US 657911**

(43) Date of publication of application:
**09.04.86 Bulletin 86/15**

(45) Publication of the grant of the patent:
**30.09.92 Bulletin 92/40**

(84) Designated Contracting States:
**DE FR GB IT**

(56) References cited:
**DE-A- 2 314 509**
**US-A- 3 996 934**

**H. MARK et al.: "ENCYCLOPEDIA OF POLY-MER SCIENCE AND TECHNOLOGY; Plastics, Resins, Rubbers, Fibers", vol. 10, 1969, J. Wiley, New York, US; page 269**

**JAPANESE PATENTS GAZETTE, Section Ch: Chemical, Week E37, 1982, ref. no. 77553, Derwent Publications, London, GB; & JP-A-57 126 416 (SEKISUI CHEMI IND K.K.) 06-08-1982**

(73) Proprietor: **HERCON LABORATORIES CORPO-RATION**
**1212 Avenue of the Americas**
**New York, New York 10036(US)**

(72) Inventor: **Kydonieus, Agis F.**
**1409 Second Avenue**
**New York New York 10021(US)**
Inventor: **Kauffman, Donald E.**
**511 Pennsylvania Avenue**
**York Pennsylvania 17404(US)**

(74) Representative: **Woodcraft, David Charles et al**
**BROOKES & MARTIN High Holborn House**
**52/54 High Holborn**
**London, WC1V 6SE(GB)**

## Description

This invention relates broadly to devices for the controlled release and transdermal administration of nitroglycerin in the treatment of conditions, e.g., angina pectoris.

Many prior art devices have been disclosed for the transdermal delivery of various drugs including nitroglycerin. In general, however, due to the potentially explosive property of nitroglycerin, available commercial devices have been generally restricted to a low concentration of the drug. One such device, for example, contains nitroglycerin in water microdispersed in a silicone oil to give a 2% nitroglycerin concentration; another device uses a microporous polymeric matrix filled with a low concentration of nitroglycerin.

Prior art devices for transdermal administration of nitroglycerin also generally require that the transdermal bandage have a large surface area; because of the relatively low concentration of nitroglycerin in the available devices, relatively large areas of the patient's skin must be contacted in order to achieve a desired rate of administration. This may contribute to the patient's discomfort and inconvenience.

Canadian Patent No. 930,668 discloses a bandage for administering drugs comprised of a backing member, a pressure sensitive adhesive, and at least one reservoir disposed between the backing member and pressure sensitive adhesive. The reservoir is comprised of a systemically active drug formulation confined within a wall member, the wall member being formed from a drug release rate controlling material. The reservoir can be in the form of discrete microcapsules or distinct reservoir compartments or layers. The reservoir can also be in the form of walled containers having one or more interior drug-containing chambers, as well as solid matrixes having a systemically active drug distributed therethrough. The Canadian patent discloses a wide variety of materials which can be used to form the reservoir. Among the materials mentioned are silicone rubbers, hydrophilic polymers of monoesters of an olefinic acid, polyvinylalcohol, polyvinylacetate, plasticized polyvinylchloride, plasticized nylon, collagen, modified collagen, gelatin, and waxes such as polyethylene wax, oxidized polyethylene wax, hydrogenated castor oil and the like, with the silicone rubbers being preferred. The Canadian patent does not contain any examples showing the use of plasticized polyvinyl chloride, and does not show the use of a PVC plastisol.

As is well known, polyvinyl chloride (PVC) is never used alone, but is always mixed with other ingredients before being processed. Polyvinyl chloride appeared at first to be an unpromising resin because it is insoluble in common solvents, cannot be molded without thermal decomposition and turns black in a few days exposure to sunlight. PVC, however, was discovered to form a rubberlike material when dissolved hot in high boiling solvents known as plasticizers and cooled to room temperature. PVC is now available in a number of different physical forms and types, and its manufacture depends on the form desired. Thus, PVC is available as a vinyl latex, a dispersion resin, or a general purpose resin. PVC latexes are true colloidal dispersions of submicrometer particles in water, stabilized by a surfactant system, and need plasticizers in order to form a continuous film. The PVC in vinyl latex is manufactured by emulsion polymerization.

Dispersion resins are produced by emulsion polymerization and are mixed with plasticizers to form a colloidal dispersion. Such dispersions are known as plastisols and are easily handled and readily pourable. When heated to a temperature of 148 to 177°C, the plastisol is transformed to a homogenous melt which, upon cooling to below 50°C, results in a tough flexible product. The PVC resins made by emulsion polymerization are hard spheres of particle size between 0.05 and 20 $\mu$m, such as between 1 and 20 $\mu$m. They do not have the ability to absorb plasticizers. Therefore, a mixture containing, for example, 30% plasticizer and 70 PVC resin, produces a flowable liquid, known as plastisol.

General purpose PVC resins are made by mass and suspension polymerization process, and comprise the largest amount of PVC resins produced, such as at least 80 percent of all PVC resins, and are used chiefly to make so-called 100% vinyl products by a variety of molding and extrusion techniques. Resins intended for flexible applications should have good uptake of plasticizer in a dry blending operation and contain more than 25% of a plasticizer system. PVC compounds that contain less than 25% plasticizers are referred to as semirigid compounds. The PVC resins manufactured by suspension and bulk polymerization are 50 to 200, such as 100 to 150 microns in diameter, and are like sponges. They are capable of absorbing large amounts of plasticizers, so that even a 50% plasticizer, 50% PVC resin composition would result in a non-flowing, solid material.

## SUMMARY OF THE INVENTION

It is an object of the present invention to provide a device which offers enhanced delivery capability over prior commercial devices by providing a device capable of safely administering nitroglycerin directly from a plastisol resin layer in which the nitroglycerin is incorporated in relatively high concentration.

This invention also enables the transdermal administration of nitroglycerin to be achieved through a device requiring contact with a relatively smaller area of the patient's skin.

Additional objects and advantages of the present invention will be set forth in part in the description which follows and in part will be obvious from the description or can be learned by practice of the invention. The objects and advantages are achieved by means of the products, instrumentalities and combinations particularly pointed out in the appended claims.

This invention is directed to a device incorporating a polyvinyl chloride resin plastisol monolayer for a transdermal administration of nitroglycerin to a patient requiring treatment for a condition, such as, angina pectoris. In particular, it has been found that nitroglycerin can be homogeneously dispersed in high concentrations in a plastisol of an emulsion polymerized polyvinyl chloride resin and plasticizer; the composition is fused into a solid layer and the layer is applied on the skin of a patient whereby the nitroglycerin is absorbed through the patient's skin into the circulation system. A preferred embodiment comprises a multilayer adhesive bandage device incorporating a plastisol monolayer containing homogeneously dispersed nitroglycerin for the treatment of angina.

Brief Description of the Drawings

Figure 1 is a cross-sectional view of a preferred nitroglycerin delivery device in accordance with this invention;
Figure 2 is a plan view of a strip of material in accordance with this invention viewed from the surface which is applied to the patient's skin;
Figure 3 is a plan view of material in accordance with this invention viewed from the surface away from the surface which is applied to the patient's skin, and
Figure 4 is a perspective view of a roll of material in accordance with this invention.

Detailed Description of the Invention

The invention is described herein with respect to a preferred embodiment useful in the treatment of angina pectoris with nitroglycerin, but it will be understood that other structural embodiments may be employed within the spirit of this invention.

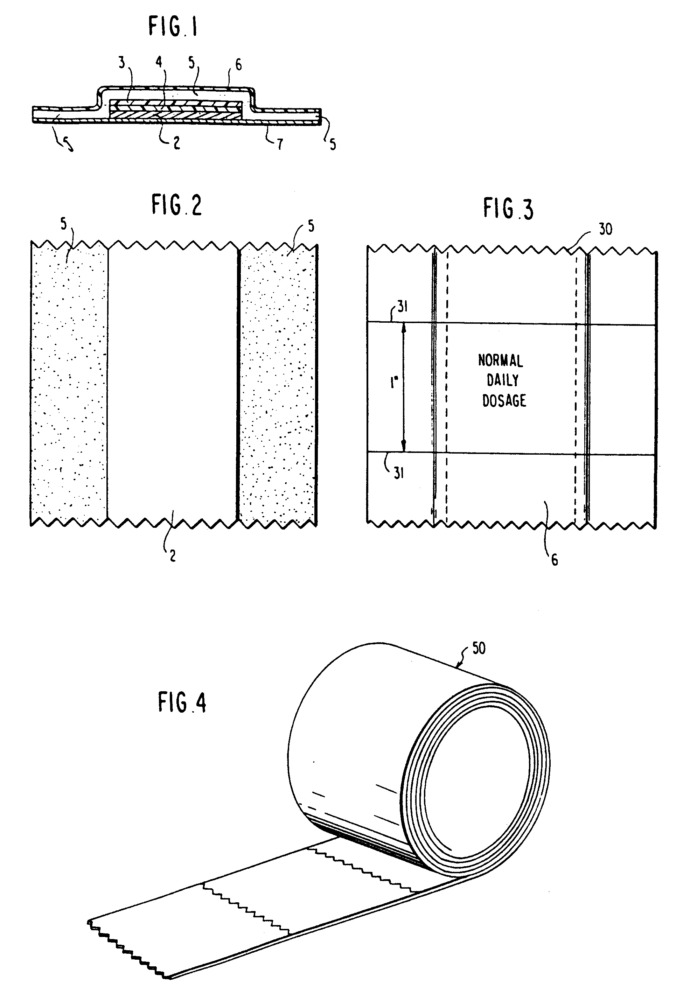

Referring to Figure 1, the device 1 includes a solid plastisol monolayer 2 which is composed of or comprises resin, plasticizer, and nitroglycerin.

It has been found that a safe, i.e., non-explosive formulation for layer 2 generally may comprise from 40 to 70% vinyl resin, 25 to 45% plasticizer and the balance nitroglycerin.

For a device suitable in the treatment of angina pectoris, a specific formulation for layer 2 comprises about 54% polyvinylchloride (PVC) resin, about 36% dioctyl phthalate (DOP) plasticizer, and 10% nitroglycerin. To prepare layer 2, the PVC and DOP are first blended; due to the explosive character of the drug, dispersion of the nitroglycerin, not encapsulated, into the resulting PVC/DOP plastisol is then carried out under controlled conditions by a subcontractor in a facility equipped with the necessary apparatus for handling nitroglycerin. The resulting plastisol blend is no longer hazardous.

In addition to DOP, other useful plasticizers include benzylbutylphthalate and tri-2-ethylexylmaleate.

The PVC resin employed in the practice of the present invention is that which is specifically used in preparing PVC plastisols, namely PVC resins which are made by the well known emulsion polymerization process, which are hard spheres of particle size between 0.05 and 20 $\mu$m, such as between 1 and 20 $\mu$m, or between 0.05 and 1 $\mu$m, and which, do not have the ability to absorb plasticizers to any great extent. Instead, the plasticizer wets the resin particles at room temperature and only very slowly penetrates and solvates the resin. These PVC resins, when mixed with plasticizers, such as a mixture of 30% plasticizer-70% PVC resin, give a flowable liquid, known as plastisol which can then be fused at, for example, 121°C (250°F) for 30 seconds, to give a useful solid polymer layer. The PVC resin employed in the plastisol layer of the present invention is in contrast to the general purpose PVC resins which are produced by suspension and bulk polymerization and which are used in calendering and extrusion process, which are 50 to 200 $\mu$m, such as 100 to 150 $\mu$m, in diameter, and are like sponges. Thus, the general purpose resins are capable of absorbing large amounts of plasticizers so that even a 50% DOP and 50% PVC resin would result in a non-flowing, solid material.

The weight average molecular weight of the PVC resins employed in the present invention is between 100,000 and 200,000. contrast to the general purpose PVC resins which are produced by suspension and bulk polymerization and which are used in calendering and extrusion process, which are 50 to 200 microns, such as 100 to 150 microns, in diameter, and are like sponges. Thus, the general purpose resins are capable of absorbing large amounts of plasticizers so that even a 50% DOP and 50% PVC resin would result in a non-flowing, solild material.

The weight average molecular weight of the PVC resins employed in the present invention preferably is between 100,000 and 200,000.

The blended plastisol containing, for example, PVC, DOP and nitroglycerin is then coated at a rate of about 1.22 kgm$^{-2}$ (36 ounces/yd$^2$) on a backing, and then fused into solid plastisol layer 2. The backing may be a single layer of drug impermeable plastic or other material, but is preferably composed of two layers 3 and 4. Layer 3 may be MYLAR, about 13um (0.5 mils) thick, and layer 4 may be PVC, about 101 um (4 mils) thick. The backing 3, 4 substantially blocks loss of drug from the plastisol layer 2 other than in the direction of the surface which, in use, will contact the patient's skin.

The blended plastisol which is coated on the backing can be fused into a homogeneous solid by heating it for a short period, such as 15 to 30 seconds, at a tempeature of, for example, 121 to 138°C (250 to 280°F). The use of a plastisol to form solid layer 2 enables layer 2 to be formed by using a low temperature for a short period of time, and provides conditions which do not affect the stability of the drugs.

A strip of solid plastisol layer 2 and backing 3, 4 is then bonded to a pressure-sensitive adhesive layer 5 which in turn is provided with a non-adhesive backing 6 such as one made of plastic, moisture-proof fabric, aluminum foil, etc.

When not in use, the entire surface intended for skin contact is preferably covered with a release paper 7 or the like which is removed to expose surfaces of the adhesive layer 5 and drug containing plastisol layer 2 for application to the patient's skin.

Figure 2 shows a plan view of a strip of material 20 during the stage of manufacture at which a strip of the plastisol 2 (backing 3, 4 not shown) has been applied to the adhesive tape (backing 6 not shown). For the preferred device for the controlled administration of nitroglycerin, a plastisol strip preferably about 25mm (1") in width is applied on a 63 mm (2 1/2") wide pressure-sensitive adhesive strip.

Figure 3 shows a plan view of a strip of the material 30 in accordance with this invention; spaced lines 31 may be embossed or printed on the surface away from skin contact so that the patient may conveniently measure out and cut off the proper amount of tape device to provide the prescribed daily dosage. For a device for administering nitroglycerin, for example, a segment about 25 mm (1") long cut from the longer tape (resulting in an approximately 6.45 cm$^2$ (1 sq. in.) of active surface against the pateint's skin) will provide a dosage of about 17 mg/24 hours; this is believed to be an enhanced rate of delivery compared with commercially available transdermal drug delivery devices and also will provide the pateint with a bandage device having a surface area much smaller than found in previously available devices.

As shown in Figure 4, the device of the invention may conveniently be provided in the form of a tape roll 50 from which daily dosage requirements may be clipped by the patient.

The device is capable of application to humans or other animals capable of usefully absorbing drugs through the skin.

Example

A plastisol was formed by mixing 54% of a polyvinyl chloride rsin sold under the designation FPC 6338 by Occidental Chemical Corp. and having a particle size between 1 and 5 microns, 36% DOP, and 10% nitroglycerin. the product was coated onto 25um (1 mil) polyester (MYLAR) film and cured at 138°C (280°F). The final product consisting of 8.8% nitroglycerin, 31.8% DOP, 47.8% FPC 6883, 11.6% polyester. This product can be used for delivery of nitroglycerin percutaneously.

**Claims**

1. A device for the controlled release and transdermal delivery of nitroglycerin, characterised by a solid vinyl-chloride plastisol layer comprising by weight 40-70% of an emulsion polymerised polyvinyl chloride resin having a molecular weight in the range of 100,000 to 200,000, 25-45% plasticizer and 5-29% nitroglycerin which, when a surface of said layer is in contact with the skin of an animal to be treated with said nitroglycerin, is absorbed into and through skin and thence into the circulatory system of said animal, said nitroglycerin being uniformly dispersed in said plastisol layer in an amount such that an effective amount of the nitroglycerin is absorbed through the skin of said animal in a prescribed period of time, a backing layer covering the surface of said plastisol layer opposite the skin-contacting surface of said plastisol layer, said backing layer forming a barrier substantially blocking release of said nitroglycerin from the surface of said plastisol layer opposite the skin contacting surface, and means for maintaining the skin contacting surface of said plastisol layer in contact with the skin of said animal so that transdermal absorption of said nitroglycerin takes place.

2. A device according to Claim 1 , characterised in that the plasticizer is dioctyl phthalate.

3. A device according to Claim 2, characterised in that said polyvinyl chloride is present in an amount of 50-60% said dioctyl phthalate is present in amount of 30-40%, and said nitroglycerin is present in an amount of 5-20%.

4. A device according to Claim 1, 2 or 3, characterised in that the nitroglycerin is present in an amount of 10-15%.

5. A device according to any preceding claim, characterised in that it is in the form of an elongated tape having a realtively narrower strip of said plastiol layer bonded lenghtwise to a relatively wider strip of pressure-sensitive adhesive tape, the areas of said adhesive tape extending beyond the edges of said plastisol strip providing said adhesive means for adhering said device to the skin.

6. The device of claim 5, wherein said tape is provided with indicia indicating the length of a segment of said tape which is adequate to administer a prescribed dosage of said nitroglycerin.

7. The device of claim 5, in the form of a roll of tape of sufficient length to provide multiple tape segments adequate to provide a prescribed dosage of said drug.

8. The device of claim 8, wherein said roll of tape is provided with indicia indicating the length of a segment of said tape which is adequate to administer a prescribed dosage of said nitroglycerin.

**Patentansprüche**

1. Vorrichtung zur kontrollierten Freisetzung und transdermalen Abgabe von Nitroglycerin, gekennzeichnet durch eine feste Vinylchloridplastisolschicht, die 40-70 Gew.-% eines emulsionspolymerisierten Polyvinylchloridharzes mit einem Molekulargewicht im Bereich von 100.000-200.000, 25-45 Gew.-% Weichmacher und 5-29 Gew.-% Nitroglycerin aufweist, das, wenn eine Oberfläche der Schicht mit der Haut eines mit dem Nitroglycerin zu behandelnden Tieres in Kontakt liegt, in und durch die Haut und von dort in das Kreislaufsystem des Tieres absorbiert wird, wobei das Nitroglycerin in der Plastisolschicht in einer solchen Menge gleichmäßig dispergiert ist, daß innerhalb eines vorgeschriebenen Zeitraums eine wirksame Menge des Nitroglycerins durch die Haut des Tieres absorbiert wird, durch eine Rückschicht, die die Oberfläche der Plastisolschicht bedeckt, die zu der mit der Haut in Kontakt befindlichen Oberfläche der Plastisolschicht entgegengesetzt ist, wobei die Rückschicht eine Barriere bildet, die die Freisetzung des Nitroglycerins von der Oberfläche der Plastisolschicht, die zu der mit der Haut in Kontakt befindlichen Oberfläche entgegengesetzt ist, im wesentlichen blockiert, und durch eine Einrichtung, um die mit der Haut in Kontakt befindliche Oberfläche der Plastisolschicht in Kontakt mit der Haut des Tieres zu halten, so daß eine transdermale Absorption des Nitroglycerins stattfindet.

2. Vorrichtung nach Anspruch 1, dadurch gekennzeichnet, daß der Weichmacher Dioctylphthalat ist.

3. Vorrichtung nach Anspruch 2, dadurch gekennzeichnet, daß das Polyvinylchlorid in einer Menge von 50-60 %, das Dioctylphthalat in einer Menge von 30-40 % und das Nitroglycerin in einer Menge von 5-20 % vorhanden ist.

4. Vorrichtung nach Anspruch 1, 2 oder 3, dadurch gekennzeichnet, daß das Nitroglycerin in einer Menge von 10-15 % vorhanden ist.

5. Vorrichtung nach einem der vorhergehenden Ansprüche, dadurch gekennzeichnet, daß sie in Form eines langen Bands ausgebildet ist, bei dem ein relativ schmalerer Streifen der Plastisolschicht in Längsrichtung mit einem relativ breiteren Streifen von Selbstklebeband verbunden ist, wobei die Flächen des Klebebands über die Ränder des Plastisolstreifens hinaus verlaufen unter Bildung der Hafteinrichtung zum Aufkleben der Vorrichtung auf die Haut.

6. Vorrichtung nach Anspruch 5, wobei das Band mit Markierungen versehen ist, die die Länge eines Bandsegments anzeigen, die zur Verabreichung einer vorbestimmten Dosis des Nitroglycerins ausreichend ist.

7. Vorrichtung nach Anspruch 5 in Form einer Bandrolle ausreichender Länge, um eine Vielzahl von Bandsegmenten bereitzustellen, die ausreichen, um eine vorgeschriebene Dosis des Medikaments zu liefern.

8. Vorrichtung nach Anspruch 7, wobei die Bandrolle mit Markierungen versehen ist, die die Länge eines Bandsegments anzeigen, die ausreicht, um eine vorgeschriebene Dosis des Nitroglycerins zu verabreichen.

**Revendications**

1. Dispositif pour le relâchement contrôlé et la libération transdermique de nitroglycérine, caractérisé par une couche de plastisol de chlorure de vinyle solide comprenant 40 à 70% en poids d'une émulsion de résine de chlorure de polyvinyle polymérisé ayant une masse moléculaire dans le domaine de 100,000 à 200,000, 25 à 45% de plastifiant et 5 à 29% de nitroglycérine qui, lorsqu'une surface de ladite couche est en contact avec la peau d'un animal devant être traité avec ladite nitroglycérine, est absorbée dans et au travers de la peau et ainsi qu'au sein du système circulatoire dudit animal, ladite nitroglycérine étant dispersée uniformément dans ladite couche de plastisol dans une quantité telle qu'une quantité effective de nitroglycérine est absorbée au travers de la peau dudit animal durant une durée prédéterminée, une couche de support couvrant la surface de ladite couche de plastisol du côté opposé à la surface de ladite couche de plastisol en contact avec la peau, ladite couche de support constituant une barrière empêchant de façon substantielle le relâchement de ladite nitroglycérine à partir de la surface de ladite couche de plastisol se trouvant du côté opposé à la surface en contact avec la peau, et des moyens pour maintenir la surface en contact avec la peau de ladite couche de plastisol en contact avec la peau dudit animal de sorte que l'absorption transdermique de ladite nitroglycérine a lieu.

2. Dispositif selon la revendication 1, caractérisé en ce que le plastifiant est du phtalate de dioctyle.

3. Dispositif selon la revendication 2, caractérisé en ce que ledit chlorure de polyvinyle est présent dans une quantité de 50 à 60%, ledit phtalate de dioctyle est présent dans une quantité de 30 à 40% et ladite nitroglycérine est présente dans une quantité de 5 à 20%.

4. Dispositif selon les revendications 1, 2 ou 3, caractérisé en ce que la nitroglycérine est présente dans une quantité de 10 à 15%.

5. Dispositif selon l'une quelconque des revendications précédentes, caractérisé en ce qu'il a la forme d'un ruban allongé ayant une bande relativement plus étroite de ladite couche de plastisol collée dans le sens de la longueur à une bande relativement plus large d'adhésif sensible à la pression, les zones de ladite bande d'adhésif s'étendant au-delà des côtés de la couche de plastisol constituant des moyens adhésifs pour faire adhérer ledit dispositif à la peau.

6. Dispositif selon la revendication 5, caractérisé en ce que ledit ruban est pourvu de signes indiquant la longueur d'un segment dudit ruban qui est adapté pour administrer une dose prédéterminée de ladite nitroglycérine.

7. Dispositif selon la revendication 5, sous forme d'un rouleau de bande d'une longueur suffisante pour fournir plusieurs segments de bande adaptés à donner une dose prédéterminée de ladite drogue.

8. Dispositif selon la revendication 7, caractérisé en ce que ledit rouleau de bande est pourvu de signes indiquant la longueur d'un segment de ladite bande qui est adapté pour administrer une dose prédéterminée de ladite nitroglycérine.

**FIG.1**

**FIG.2**

**FIG.3**

NORMAL
DAILY
DOSAGE

**FIG.4**